# EUROPEAN PATENT SPECIFICATION

(11) **EP 3 954 422 B1**
(45) Date of publication and mention of the grant of the patent: **15.05.2024**
(21) Application number: 21199981.8
(22) Date of filing: 18.01.2018
(51) Int. Cl.: A61M 16/08, A61M 39/10, A61M 39/12, F16L 33/00, A61M 16/06

(54) **PATIENT INTERFACE CONNECTOR**
PATIENTENSCHNITTSTELLENVERBINDER
CONNECTEUR D'INTERFACE DE PATIENT

(30) Priority: 20.01.2017 GB 201701042
(43) Date of publication of application: 16.02.2022
(62) Divisional of application: 18702152.2
(73) Proprietor: Intersurgical AG, 9490 Vaduz (LI)
(72) Inventor: BOWSHER, Richard Francis, Wokingham, Berkshire RG41 2RZ (GB)
(74) Representative: Adamson Jones

(56) References cited:
- WO-A1-2014/124323
- WO-A1-2015/033263
- US-A1- 2009 260 628
- US-A1- 2014 261 433
- US-B2- 7 174 893

## Description

The present invention relates to a patient interface connector for connecting a patient interface to a conduit.

Oxygen delivery masks are used to supply a patient with oxygen, to aid their normal respiratory function. In general, oxygen is supplied to a respiratory enclosure defined by the oxygen delivery mask and the face of the patient, and the patient inhales the oxygen from this respiratory enclosure, sometimes along with ambient gases from outside the respiratory enclosure. Conventional oxygen delivery masks typically have an inlet for the oxygen, as well as inhalation and exhalation outlets through which ambient gases and exhaled gases can pass.

To connect a supply of oxygen to the inlet of the mask, it is typical to utilise a connector which is connected to the inlet of the oxygen delivery mask via a friction fit connection. Whilst this can provide an adequate connection between the oxygen delivery mask and the supply of oxygen, such connections can lead to misconnection of other forms of medical tubing, for example feeding tubing, to the oxygen delivery mask, and there is also the risk of a loose connection and the potential disconnection of the supply of oxygen during normal use.

WO 2015/033263 discloses an elbow connector, for use with a patient interface device, that includes a coupling portion structured to be inserted into an opening in the patient interface device to couple the elbow connector with the patient interface device. The coupling portion includes an upper coupling portion and a lower coupling portion. The elbow connector also includes a rear surface. Applying pressure to the rear surface causes the upper coupling portion to move towards the lower coupling portion.

There has now been devised a patient interface, a patient interface connector, a patient interface assembly, and a kit, which overcome or substantially mitigate the aforementioned and/or other disadvantages associated with the prior art.

According to a first aspect of the present invention there is provided a patient interface assembly as defined in the appended claim 1. Further optional features are recited in the associated dependent claims.

Also described is a kit comprising a patient interface, a separate connector for connecting the patient interface to a conduit, and a conduit joined to the connector in a non-releasable manner, wherein the patient interface comprises a first connection formation and the connector comprises a second connection formation, wherein the patient interface and the connector are connectable such that, in a connected configuration, the first connection formation and the second connection formation are engageable with each other, with a snap fit, to provide a non-releasable connection.

The first connection formation may comprise a shoulder having an abutment surface, and at least a portion of the first connection formation and/or the second connection formation may be resiliently deformable to allow the second connection formation to engage the abutment surface in the connected configuration with a snap-fit.

The patient interface assembly and kit are advantageous principally as the patient interface and the connector are connectable with a snap-fit, which may provide a simpler and lower cost secure connection, ie non-releasable connection, relative to the prior art. The connection arrangement may also enable a simple and low cost rotatable connector. Furthermore, in a connected configuration, the first connection formation and the second connection formation cooperate to provide a non-releasable connection, which may reduce the risk of insecure connections relative to connections utilised in the prior art. In particular, the 'snap' of the snap-fit connection may inform the user that the connection is secure.

By non-releasable is meant that the connection cannot be undone by the applications of forces which are experienced during normal use of the assembly, and there is no mechanism for disengaging the connection formations from each other, and hence releasing the connection, without either breaking or fracturing of the first and/or second connection formation, or by application of a force to the patient interface and/or the connector that exceeds a pre-determined threshold force along an axis of engagement between the patient interface and the connector.

The connection may be non-releasable to the degree where the connection cannot be undone without causing structural damage to at least one of the first and second connection formations. Alternatively, the connection may be releasable only upon application of a force which exceeds a pre-determined threshold force, for example upon the application of a force which exceeds a pre-determined threshold force along an axis of engagement between the patient interface and the connector, which may or may not be a longitudinal axis of the conduit, eg away from the patient interface. The connection may be releasable only upon the application of a force, along an axis of engagement between the patient interface and the connector, having a magnitude which exceeds the forces experienced during normal use by a magnitude of at least 100%, at least 200%, at least 300%, at least 400%, at least 500%, at least 600%, at least 700% or at least 800%. The forces experienced during normal use are typically 10N or less. The pre-determined threshold force may therefore be at least 20N, at least 30N, at least 40N, at least 50N, at least 60N, at least 70N, at least 80N or at least 90N.

The first and second connection formations are engageable with a snap fit. The first connection formation may comprise a shoulder having an abutment surface, and at least a portion of the first connection formation and/or the second connection formation may be resiliently deformable to allow the second connection formation to engage the abutment surface in the connected configuration with a snap-fit.

A packaged kit is also described, comprising packaging and a kit as previously described, wherein the kit is contained within the packaging with the patient interface and the connector being in an unconnected configuration.

The kit may be entirely contained within the packaging, and may, for example be sealed within the packaging. The packaging may comprise an instruction manual, for example a manual containing instructions for use of the kit.

The connector may be joined to the conduit at one end thereof. The conduit may be bonded to the connector for example by adhesive bonding, solvent bonding, ultrasonic or thermal welding or the like. The conduit may be integrally formed with the connector. The conduit and the connector may be formed from a single material, for example such that the conduit and the connector form a single unitary body. The conduit and the connector may be formed as part of a single shot injection moulding process. The conduit and the connector may be formed from two or more separate materials. The connector may be overmoulded onto the conduit, for example as part of a multi-shot, insert-moulded, or co-moulded, injection moulding process.

The conduit may comprise an oxygen delivery conduit, for example a conduit which supplies oxygen to the patient interface in use.

The patient interface may comprise an oxygen delivery interface, for example an interface which provides for oxygen delivery to a patient in use. The patient interface may comprise an oxygen delivery mask, which may, for example, comprise a mask shell. The patient interface may comprise a full face mask or a nasal mask. The patient interface may comprise a sealing member, which may, for example, comprise a single walled membrane.

The mask shell may be adapted to define an internal cavity. The mask shell may be generally dome-shaped so as to define an internal cavity. The mask shell may comprise a substantially concave interior surface. The mask shell may comprise a substantially convex exterior surface.

At least a portion of the mask shell may be resiliently deformable. A region of the mask shell adjacent to a point of connection between the mask shell and the connector may be resiliently deformable. At least a portion of the mask shell adjacent to a recess or slot in the mask shell may be resiliently deformable. The mask shell may comprise polypropylene.

The mask shell may comprise at least one aperture for allowing passage of gases between the interior of the mask shell and the exterior of the mask shell. The at least one aperture may comprise an inhalation and/or exhalation aperture. The at least one aperture may be spaced apart from an oxygen inlet of the mask shell.

The patient interface protrudes outwardly to a greater extent in a nasal region of the patient interface, such that a nasal shelf is formed. The nasal shelf may be defined by a wall of the mask shell of the patient interface. The mask shell of the patient interface may comprise a single unitary component.

The connector may be shaped to provide a compression force to the conduit, for example to retain the connector in position relative to the conduit. The connector may comprise an internal cross-sectional shape which deforms the conduit when the connector and the conduit are connected. The connector may comprise a polygonal internal cross-sectional shape, and may, for example, comprise a polygonal internal cross-sectional shape having curved edges, for example convex curved edges. The connector may comprise a substantially constant internal diameter, and the interior of the connector may, for example comprise a substantially constant cross-sectional area and/or a substantially constant internal cross-sectional shape. The interior of the connector may comprise a plurality of different cross-sectional areas, with a transition between cross-sectional areas of a different shape. The interior of the connector may comprise a region having a circular cross-sectional area, and a region having a polygonal cross-sectional area, with a transitional region therebetween. The connector may comprise an internal diameter corresponding substantially to the external diameter of the conduit. The connector may comprise an external and/or internal diameter in the region of 3mm to 10mm. The polygonal cross-sectional area may be substantially in the shape of a reuleaux triangle.

The first connection formation comprises a slot formed in an outer surface of the patient interface. Such a configuration may be beneficial as this may reduce the complexity of the manufacturing process of the patient interface. For example, such a configuration may remove the need for moving cores in the tool for moulding the patient interface. The tool may be a so-called "open and shut" tool, and may mean that more cavities can be included in the tool to make the patient interface. This may reduce the cycle time for the moulding of the patient interface, and may allow for more shots per cycle.

The slot is configured to receive the second connection formation in a direction which is substantially orthogonal to a longitudinal axis of the conduit. An axis of insertion of the connector into the recess is substantially orthogonal to a longitudinal axis of the conduit. An axis of insertion of the second connection formation is substantially orthogonal to, a longitudinal axis of the conduit.

The slot extends in a direction which is substantially orthogonal to a longitudinal axis of the conduit when the patient interface and the connector are in a connected configuration. The slot extends in a direction which requires insertion of the second connection formation into the slot in a direction which is substantially orthogonal to a longitudinal axis of the conduit. The slot may extend along a direction of a nasal shelf of the patient interface. The slot may, for example, extend into the nasal shelf, such that at least a portion of the nasal shelf defines a nasal shelf aperture for receiving at least a portion of the connector in a connected configuration. The nasal shelf aperture may have a width, for example a diameter, that is less than a corresponding width of the slot of the first connection formation.

The slot is defined by at least one internal wall of the patient interface and a nasal shelf wall of the patient interface. The at least one internal wall of the patient interface may prevent removal of the connector from the recess in a direction substantially orthogonal to a longitudinal axis of the conduit in the connected configuration. The nasal shelf wall of the patient interface may prevent removal of the connector from the recess in a direction substantially parallel to a longitudinal axis of the conduit in the connected configuration.

The slot may comprise the first connection formation, and the connector may comprise an enlarged head defining the second connection formation, and at least a portion of the first connection formation may be resiliently deformable to allow the second connection formation to engage the abutment surface in the connected configuration with a snap-fit.

The shoulder of the first connection formation may be located internally of the patient interface, and may for example, be formed on wall which at least partially defines the recess of the first connection formation. The shoulder may comprise an annular shoulder. Substantially the entirety of the perimeter of the annular shoulder may engage a corresponding portion of the second connection formation in a connected configuration.

At least a portion of the patient interface may resiliently deform to allow insertion of the connector into the recess, and may resiliently deform into its original shape once the connector has been fully inserted into the recess. The shoulder may prevent removal of the connector from the recess in a direction substantially orthogonal to a longitudinal axis of the conduit in the connected configuration.

The first and second connection formations may comprise a plurality of corresponding pairs abutment surfaces which contact each other in use. A first pair of corresponding abutment surfaces may lie substantially orthogonally to a longitudinal axis of the conduit in the connected configuration, whilst a second pair of corresponding abutment surfaces may lie substantially parallel to a longitudinal axis of the conduit in the connected configuration. Thus the first and second connection formations may resist disconnection in more than one direction, when in the connected configuration.

The second connection formation may comprise a surface, for example an external surface, of the connector. The second connection formation may comprise a surface of the connector which contacts a corresponding surface of the patient interface, for example an internal surface of the patient interface, in use.

The second connection formation may comprise a shoulder formation for engaging a corresponding shoulder formation of the first connection formation. The second connection formation may comprise at least one projection which extends, for example outwardly, from the connector and/or conduit, for example such that a shoulder formation is formed between the at least one projection and the connector and/or conduit. The at least one projection may extend into the interior of the slot of the patient interface and/or the interior of the patient interface, in use. The at least one projection may comprise an abutment surface for engaging a corresponding abutment surface of the first connection formation.

The at least one projection may extend radially outwardly from a main body of the connector. The at least one projection may extend at least partially annularly about the main body of the connector. The at least one projection may be upstanding from an end of the connector, for example an end of the connector which is distal from the end of the connector that is connected to the conduit, in use. The at least one projection may extend from a main body of the connector in a direction which is substantially parallel to a longitudinal axis of the connector.

The at least one projection may be located at an end of the connector which is distal from the end of the connector that is connected to the conduit, in use. The at least one projection may be located at an end of the connector that is inserted into the patient interface first, in use.

The second connection formation may comprise a plurality of projections which extend outwardly from the main body of the connector. Each projection may extend in substantially orthogonal directions. A first projection may extend substantially radially outwardly from a main body of the connector, whilst a second projection may be upstanding from an end of the connector, in a direction along a longitudinal axis of the connector.

The second connection formation may be integrally formed with the connector, for example as part of a single injection moulding process. The second connection formation may comprise a region of increased diameter relative to a main body of the connector. The second connection formation may comprise an enlarged head. The diameter of a main body of the connector may be substantially the same as an internal diameter of the recess in the patient interface, for example such that an external surface of the main body of the connector is substantially in contact with an internal surface of the recess in the patient interface, in use.

The at least one projection may comprise a substantially triangular cross-section, for example a cross-section taken along a central longitudinal axis of the connector. The at least one projection may comprise an angled leading edge, which may, for example, be acutely angled relative to the main body of the connector when the angle is measured between orthogonal longitudinal and transverse axes of the connector in a direction orthogonal to that of insertion of the connector. The at least one projection may define a holding barb at an end of the connector, for example the end of the connector which is inserted first into the recess in the patient interface, in use.

The connector may be rotatably connected to the patient interface in use, for example such that the connector is rotatable relative to the patient interface, and vice versa, in use. The first and second connection formations may be rotatably connected, in use. The abutment surfaces of the first and second connection formations may enable relative rotational movement therebetween. The connector may be rotatable about a longitudinal axis of the connector, for example a central longitudinal axis of the connector, in use.

The first and second connection formations are connected by a snap-fit, in use. For example, the second connection formation may deform upon insertion of the connector into the spigot, and the second connection formation may regain its original shape once it has passed through the internal diameter of the spigot, such that the first and second connection formations are in engagement.

The first and/or second connection formation may be configured to resist resilient deformation upon attempted removal of the connector from the patient interface, such that the connector cannot be removed from the patient interface, or vice versa, unless the removal force applied is greater than forces which are experienced during normal operating conditions, for example unless the removal force is greater than a pre-determined threshold force. The pre-determined threshold force may be at least 100%, at least 200%, at least 300%, at least 400%, at least 500%, at least 600%, at least 700%, or at least 800% greater than forces experiences during normal use. The pre-determined threshold may therefore be at least 20N, at least 30N, at least 40N, at least 50N, at least 60N, at least 70N, at least 80N or at least 90N. Alternatively, the first and/or second connection formation may be configured to resist resilient deformation upon attempted removal of the connector from the patient interface, such that the connector cannot be removed from the patient interface without causing structural breakage of the connector and/or patient interface. The first and second connection formations may be in engagement, and may be disposed substantially orthogonally relative to a direction of attempted removal of the connector from the patient interface, in the connected configuration, such that the connector cannot be removed from the patient interface, or vice versa, without causing damage to the patient interface and/or the connector. The first and/or second connection formations may form an acute and/or perpendicular angle relative to the main body of the connector in the connected configuration, the angle being measured between the main body of the connector and an axis of insertion of the connector, in a direction of insertion of the connector.

The first and/or second stop formations may comprise corresponding projections and/or recesses, for example corresponding annular projections and/or recesses. The first stop formation may comprise an annular recess formed on an internal surface of the spigot, and the second stop formation may comprise an annular projection formed on an external surface of the connector. The second stop formation may fit substantially within the first stop formation, in use. The second stop formation and the second connection formation may be located at opposing ends of the connector. The first stop formation may comprise an end surface of the spigot, and the second stop formation may comprise an annular projection formed on an external surface of the connector. The second stop formation may abut substantially the entirety of the end surface of the spigot, in use.

The patient interface may comprise a rigid patient interface, for example a patient interface with a relatively low degree of flexibility which maintains its shape when subjected to normal handling conditions. The patient interface may have a Shore A hardness greater than the Shore A hardness of the conduit. The patient interface may be formed from plastics material, for example using an injection moulding process. The patient interface may comprise polypropylene.

The connector may comprise a rigid connector, for example a connector with a relatively low degree of flexibility which maintains its shape when subjected to normal handling conditions. The connector may have a Shore A hardness greater than the Shore A hardness of the conduit. The connector may be formed from plastics material, for example using an injection moulding process. The connector may comprise polyvinylchloride (PVC), for example rigid PVC.

Practicable embodiments will now be described with reference to the accompanying drawings, of which:
Figure 1 is a perspective view of a patient interface assembly not in accordance with the present invention;
Figure 2 is an enlarged cross-sectional view of the connection interface of the patient interface assembly of Figure 1;
Figure 3 is a perspective view of the connector utilised in Figures 1 and 2;
Figure 4 is a cross-sectional view of a first alternative embodiment of a connector for use in the patient interface assembly of Figure 1;
Figure 5 is a perspective view of a second alternative embodiment of a connector for use in the patient interface assembly of Figure 1;
Figure 6 is a perspective view of a third alternative embodiment of a connector for use in the patient interface assembly of Figure 1;
Figure 7 is a cross-sectional view of a fourth alternative embodiment of a connector for use in the patient interface assembly of Figure 1;
Figure 8 is an enlarged perspective view of a patient interface assembly according to the invention;
Figure 9 is a first enlarged cross-sectional view of the connection interface of the patient interface assembly of Figure 8; and
Figure 10 is a first enlarged cross-sectional view of the connection interface of the patient interface assembly of Figure 8.

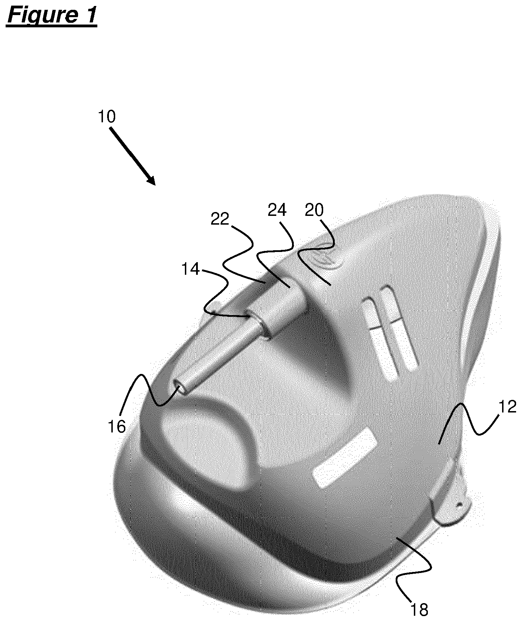

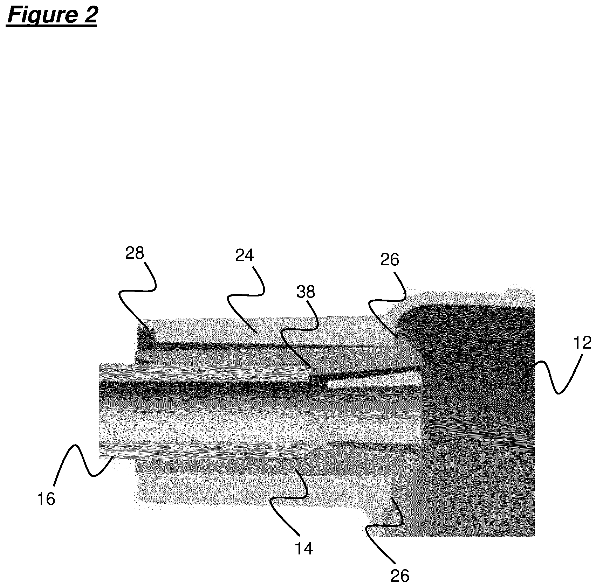

A patient interface assembly, generally designated 10, is shown in Figures 1 and 2.

The patient interface assembly 10 comprises an oxygen delivery mask 12, a connector 14, and an oxygen conduit 16.

The oxygen delivery mask 12 comprises a rigid mask body 18, formed of polypropylene. The mask body 18 is generally concave in from, and defines an internal cavity. A nasal region 20 of the mask body 18 extends forwardly of the remainder of the mask body 18, thereby defining a shelf 22. A female spigot 24 depends from the shelf 22.

The female spigot 24 is substantially cylindrical in global form, and has a hollow interior. The internal diameter of the female spigot 24 is substantially the same is the external diameter of the main body of the connector 14. A first end of the female spigot 24 defines an internal annular shoulder 26, whilst a second end of the female spigot 24 defines an internal annular recess 28.

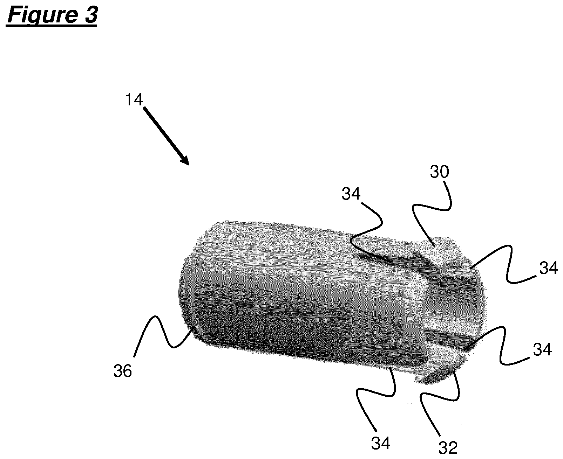

The connector 14 is shown in more detail in Figures 2 and 3. The connector 14 has a substantially cylindrical global form, and is hollow. The connector 14 is generally rigid in nature, and is formed of polyvinylchloride (PVC). A first end of the connector 14 comprises first 30 and second 32 projections, and a plurality of slots 34. The first 30 and second 32 projections are integrally formed with, and extend radially outwardly from, the main body of the connector 14. The first 30 and second 32 projections thereby form a region of increased diameter relative to the main body of the connector 14. Each of the plurality of slots 34 is located adjacent a corresponding projection 30,32, and extends longitudinally along the connector 14.

A second end of the connector 14 has an annular projection 36. The annular projection 36 is integrally formed with the connector 14, and is shaped and dimensioned to engage the corresponding internal annular recess 28 of the female spigot 24 in use. The connector 14 further comprises an internal abutment shoulder 38 for preventing over-insertion of a conduit 16 into the connector 14.

In use, a conduit 16 is bonded to the interior of the connector 14 using adhesive glue, such that a flow path is defined through the interior of the conduit 14. The connector 14 is inserted into the female spigot 24. The slots 34 allow the region of the connector 14 having the first 30 and second 32 projections to deform during passage of the connector 14 through the female spigot 24. Once the first 30 and second 32 projections have passed through the interior of the female spigot 24 and into the interior of the mask body 18, the first 30 and second 32 projections resiliently deform such that the connector 14 reverts to its original pre-deformation shape. In other words, the connector 14 is connected to the oxygen delivery mask 12 with a snap-fit connection

The first 30 and second 32 projections are now in contact with the internal annular shoulder 26 of the female spigot 24, whilst the annular projection 36 of the connector 14 is in contact with the internal annular recess 28 of the female spigot 24. Movement of the connector 14 along a longitudinal axis of the connector 14 is now inhibited by the engagement between the first 30 and second 32 projections and the internal annular shoulder 26, as well as by the engagement between the annular projection 36 and the internal annular recess 28. Thus the connector 14 cannot be removed from the oxygen delivery mask 12 without causing damage to either the connector 14 or the oxygen delivery mask 12. The nature of the connection still enables the connector 14 to rotate about its central longitudinal axis, which inhibits the formation of any kinks or strain in the conduit 16.

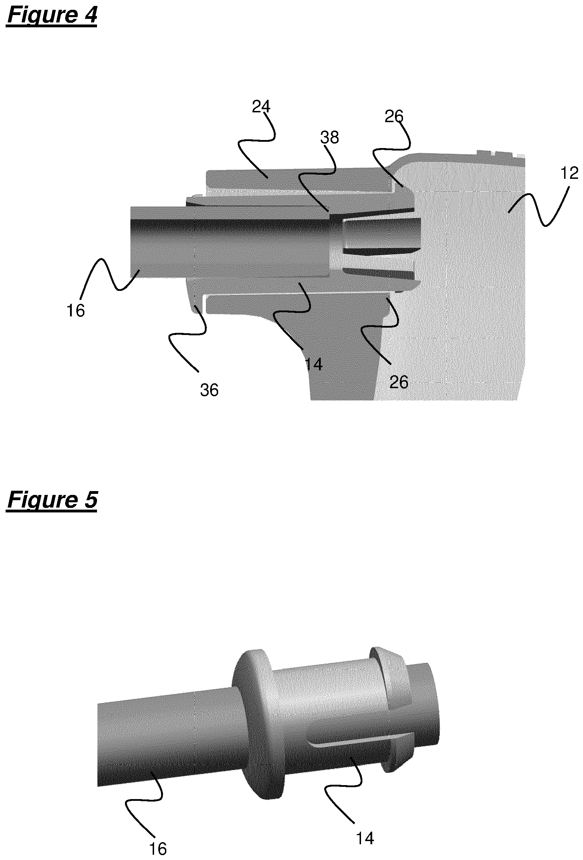

A first alternative embodiment of a connector 14 is shown in Figure 4. The embodiment of the connector 14 shown in Figure 4 is substantially the same as the embodiment of the connector 14 shown in Figures 2 and 3, and differs only in that the annular projection 36 extends outwardly from the body of the connector 14 to a much greater degree, such that the annular projection abuts the end surface of the spigot 24 in use. This prevents over insertion into the spigot 24, and removes the need for the internal annular shoulder 26 of the spigot 24.

A second alternative embodiment of a connector 14 is shown in Figure 5. The connector of Figure 5 is substantially the same as the connector of Figure 4, and differs only in that the connector 14 is overmoulded directly onto the conduit 16.

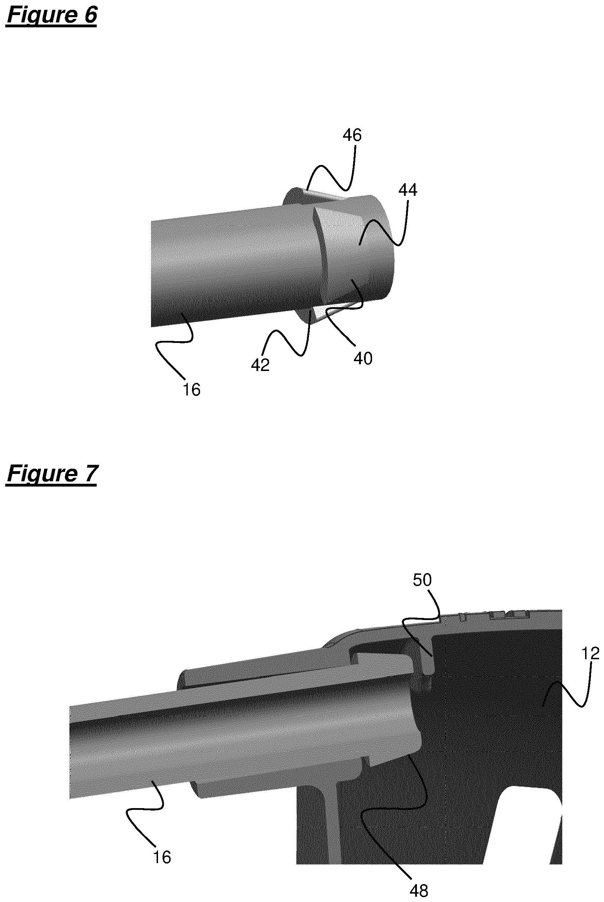

A third alternative embodiment of a connector 40 is shown in Figure 6. As in the embodiment of Figure 5, the connector 40 is over-moulded directly onto the conduit 16. The connector 40 comprises a first 42, second 44, and third 46, projections spaced circumferentially about the conduit 16. The projections 42,44,46 have a form similar to the projections 30,32 of the previously discussed connectors 14, and operate in a similar manner to retain the connector 40 relative to the oxygen delivery mask 12.

A fourth alternative embodiment of a connector 48 is shown in Figure 7, attached to an oxygen delivery mask 12. The connector 48 is integrally formed with the conduit 16 as part of a single injection moulding process, and takes the form of an annular projection extending about one end of the conduit 16 to form an enlarged head. The connector 48 operates in a similar manner to other embodiments of connectors 14, 40 previously discussed. In use, the connector 48 abuts an internal wall 50 of the oxygen delivery mask 12, to prevent over insertion of the conduit 16 into the mask 12.

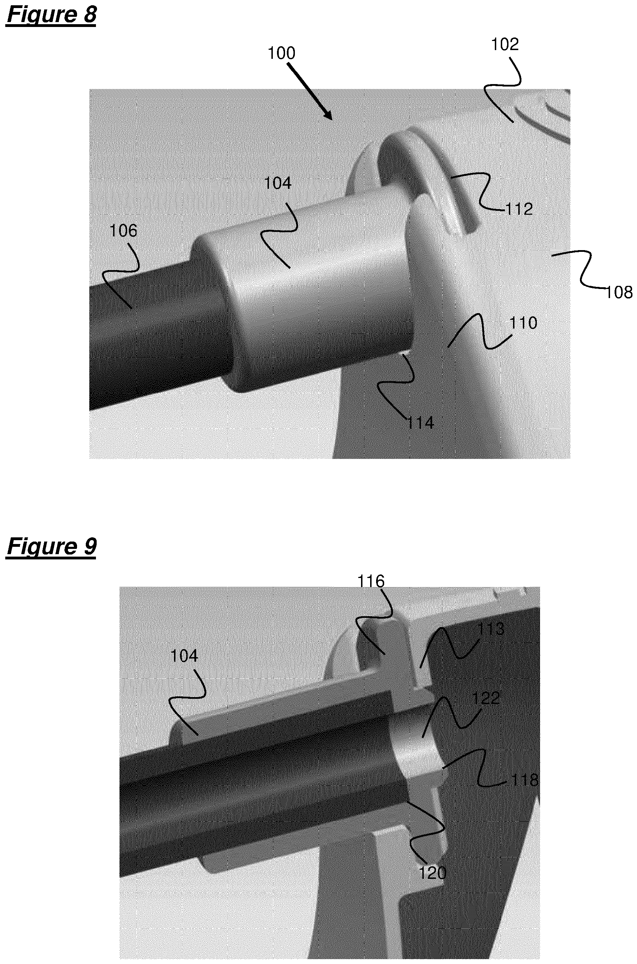

An embodiment of a patient interface assembly according to the present invention, generally designated 100, is shown in Figures 8 to 10.

The patient interface assembly 100 comprises an oxygen delivery mask 102, a connector 104, and an oxygen conduit 106.

The oxygen delivery mask 102 comprises a rigid mask body 108, formed of polypropylene. The mask body 108 is generally concave in from, and defines an internal cavity. A nasal region of the mask body 108 extends forwardly of the remainder of the mask body 108, thereby defining a shelf 110. A slot 112 is formed in the mask body 108 slightly above the shelf 110, whilst an aperture 114 is formed in the shelf 110 itself. The slot 112 has a diameter larger than the diameter of the aperture 114. The aperture 114 has a diameter corresponding substantially to the diameter of the main body of the conduit 104. The slot 112 is formed between an internal wall 113 of the mask body 108 and the shelf 110. The internal wall 113 defines a recess 115 which receives a second projection 118 of the conduit 104 in a connected configuration

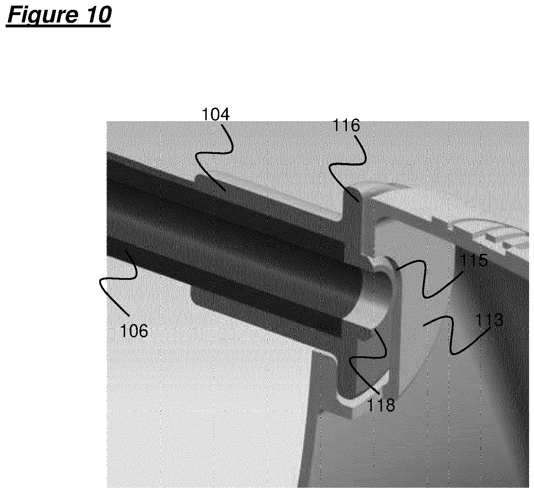

The connector 104 is shown in more detail in Figures 9 and 10. The connector 104 has a substantially cylindrical global form, and is hollow. The connector 104 is generally rigid in nature, and is formed of polyvinylchloride (PVC). A first end of the connector 104 comprises first 116 and second 118 projections. The first 116 and second 118 projections are integrally formed with the connector. The connector 104 further comprises an internal abutment shoulder 120 for preventing over-insertion of a conduit 106 into the connector 104.

The first projection 116 extends annularly about an end the main body of the connector 104, such that the first projection 116 forms an enlarged head of the connector 104 at one end thereof. The diameter of the first projection 116 is substantially the same as that of the slot 112. The second projection 118 is upstanding from the same end of the connector 104, and is substantially annular in form. The second projection 118 defines an aperture 122 which is shaped and dimensioned to correspond to the internal diameter of the conduit 106. The outer surface of the second projection 118 is shaped to define an angular upper portion, and a linear lower portion, as seen in Figure 10. The second projection 118 is resiliently deformable.

In use, a conduit 106 is bonded to the interior of the connector 104 using adhesive glue, such that a flow path is defined through the interior of the conduit 104. The connector 104 is inserted into the slot 112 via the first projection 116. When the angled upper outer surface of the second projection 118 first engages the mask body 108, the mask body 108 in the region of the slot 112 resiliently deforms to allow continued insertion of the connector 104 into the slot 112. Upon further insertion of the connector 104, the second projection 118 engages the recess 115 in the internal wall 113, and the mask body 108 returns to its original shape, such that the connector 104 engages the mask body 108 with a snap-fit. In particular, the linear lower portion of the outer surface of the second projection 118 engages the internal wall 113. The main body of the connector 104 is received within the aperture 114.

In the connected configuration, the linear lower portion of the outer surface of the second projection 118 engages the internal wall 113, to prevent removal of the connector 104 from the mask body 108 in a direction that is orthogonal to a longitudinal axis of the conduit 106. The second projection 116 engages the shelf 110 to prevent removal of the connector 104 from the mask body 108 in a direction that is parallel to a longitudinal axis of the conduit 106.

## Claims

1. A patient interface assembly (100) comprising a patient interface (108), a connector (104) for connecting the patient interface to a conduit (106), the conduit (106) being joined to the connector (104) in a non-releasable manner, the patient interface (108) comprising a first connection formation and the connector (104) comprising a second connection formation, the first connection formation comprising a slot (112) for receiving at least a portion of the second connection formation, wherein the slot (112) extends in a direction which requires insertion of the second connection formation into the slot (112) in a direction which is substantially orthogonal to a longitudinal axis of the conduit (106), the patient interface (108) and the connector (104) being connectable such that, in a connected configuration, the first connection formation and the second connection formation are engageable with each other, with a snap fit, to provide a non-releasable connection, and
wherein a nasal region of the patient interface (108) extends forwardly of the remainder of the patient interface (108), thereby defining a nasal shelf (110), and the slot (112) is formed in the mask body 108 slightly above the shelf (110), the slot (112) being defined by at least one internal wall (113) of the patient interface (108) and the nasal shelf wall (110) of the patient interface (108).

2. A patient interface assembly (100) as claimed in Claim 1, wherein the slot (112) is formed in an outer surface of the patient interface (108).

3. A patient interface assembly (100) as claimed in Claim 1 or 2, wherein the at least one internal wall (113) of the patient interface (108) prevents removal of the connector (104) from the slot (112) in a direction orthogonal to a longitudinal axis of the conduit (106) in the connected configuration, and wherein the nasal shelf wall (110) of the patient interface (108) prevents removal of the connector (104) from the slot (112) in a direction substantially parallel to a longitudinal axis of the conduit (106) in the connected configuration.

4. A patient interface assembly (100) as claimed in any preceding claim, wherein the connection is releasable only upon application of a force that exceeds a pre-determined threshold force along an axis of engagement between the patient interface (108) and the connector (104).

5. A patient interface assembly (100) as claimed in Claim 4, wherein the connection is releasable only upon the application of a force, along an axis of engagement between the patient interface (108) and the connector (104), having a magnitude at least 20N, at least 30N, at least 40N, at least 50N, at least 60N, at least 70N, at least 80N or at least 90N.

6. A patient interface assembly (100) as claimed in any preceding claim, wherein the connection is non-releasable to the degree where the connection cannot be undone without causing structural damage to at least one of the first and second connection formations.

7. A patient interface assembly (100) as claimed in Claim 4 or Claim 5, wherein the first and/or second connection formation is at least temporarily deformable upon the application of a force exceeding the pre-determined threshold, thereby disengaging the first and second connection formations and enabling release of the connection between the patient interface (108) and the connector (104), without causing structural damage to, for example breaking or fracturing of, the first and/or second connection formation.

8. A patient interface assembly (100) as claimed in any preceding claim, wherein the patient interface (108) comprises an oxygen delivery mask (102).

9. A patient interface assembly (100) as claimed in any preceding claim, wherein the first connection formation comprises at least a portion of an internal surface of the patient interface (108), and wherein the second connection formation comprises an external surface of the connector (104).

10. A patient interface assembly (100) as claimed in any preceding claim, wherein the second connection formation comprises at least one projection (116,118) which extends outwardly from a main body and/or arm of the connector (104).

11. A patient interface assembly (100) as claimed in Claim 10, wherein the at least one projection (116,118) comprises an abutment surface for engaging a corresponding abutment surface of the first connection formation, and wherein the at least one projection (116,118) extends at least partially annularly about a main body and/or arm of the connector (104).

12. A patient interface assembly (100) as claimed in any preceding claim, wherein the first and second connection formations are rotatably connected, in use.

13. A patient interface assembly (100) as claimed in any preceding claim, wherein the patient interface (108) and/or connector comprises a stop formation for preventing over insertion of the connector (104) into the patient interface (108) in use, and/or for inhibiting movement of the connector (104) relative to the patient interface (108), along a longitudinal axis of the connector (104), in use.

## Patentansprüche

1. Patientenschnittstellenanordnung (100), umfassend eine Patientenschnittstelle (108), einen Verbinder (104) zum Verbinden der Patientenschnittstelle mit einer Leitung (106), wobei die Leitung (106) mit dem Verbinder (104) in einer nicht lösbaren Weise zusammengefügt ist, wobei die Patientenschnittstelle (108) eine erste Verbindungsstruktur umfasst und der Verbinder (104) eine zweite Verbindungsstruktur umfasst, wobei die erste Verbindungsstruktur einen Schlitz (112) zum Aufnehmen mindestens eines Abschnitts der zweiten Verbindungsstruktur umfasst, wobei sich der Schlitz (112) in einer Richtung erstreckt, die ein Einführen der zweiten Verbindungsstruktur in den Schlitz (112) in einer Richtung erfordert, die im Wesentlichen orthogonal zu einer Längsachse der Leitung (106) ist, wobei die Patientenschnittstelle (108) und der Verbinder (104) derart verbindbar sind, dass in einer verbundenen Konfiguration die erste Verbindungsstruktur und die zweite Verbindungsstruktur mit einem Schnappsitz miteinander in Eingriff bringbar sind, um eine nicht lösbare Verbindung bereitzustellen, und
wobei sich ein Nasenbereich der Patientenschnittstelle (108) über den Rest der Patientenschnittstelle (108) nach vorne erstreckt, wodurch eine Nasenauflage (110) definiert wird, und der Schlitz (112) in dem Maskenkörper 108 etwas oberhalb der Auflage (110) ausgebildet ist, wobei der Schlitz (112) durch mindestens eine Innenwand (113) der Patientenschnittstelle (108) und die Nasenauflagewand (110) der Patientenschnittstelle (108) definiert ist.

2. Patientenschnittstellenanordnung (100) nach Anspruch 1, wobei der Schlitz (112) in einer äußeren Oberfläche der Patientenschnittstelle (108) ausgebildet ist.

3. Patientenschnittstellenanordnung (100) nach Anspruch 1 oder 2, wobei die mindestens eine Innenwand (113) der Patientenschnittstelle (108) eine Entfernung des Verbinders (104) aus dem Schlitz (112) in einer Richtung orthogonal zu einer Längsachse der Leitung (106) in der verbundenen Konfiguration verhindert und wobei die Nasenauflagewand (110) der Patientenschnittstelle (108) eine Entfernung des Verbinders (104) aus dem Schlitz (112) in einer Richtung im Wesentlichen parallel zu einer Längsachse der Leitung (106) in der verbundenen Konfiguration verhindert.

4. Patientenschnittstellenanordnung (100) nach einem vorhergehenden Anspruch, wobei die Verbindung nur bei Aufbringung einer Kraft, die eine vorbestimmte Schwellenkraft überschreitet, entlang einer Eingriffsachse zwischen der Patientenschnittstelle (108) und dem Verbinder (104) lösbar ist.

5. Patientenschnittstellenanordnung (100) nach Anspruch 4, wobei die Verbindung nur bei Aufbringung einer Kraft entlang einer Eingriffsachse zwischen der Patientenschnittstelle (108) und dem Verbinder (104), die eine Größe von mindestens 20 N, mindestens 30 N, mindestens 40 N, mindestens 50 N, mindestens 60 N, mindestens 70 N, mindestens 80 N oder mindestens 90 N aufweist, lösbar ist.

6. Patientenschnittstellenanordnung (100) nach einem vorhergehenden Anspruch, wobei die Verbindung in dem Maße nicht lösbar ist, in dem die Verbindung nicht getrennt werden kann, ohne eine strukturelle Beschädigung an mindestens einer von der ersten und der zweiten Verbindungsstruktur zu verursachen.

7. Patientenschnittstellenanordnung (100) nach Anspruch 4 oder Anspruch 5, wobei die erste und/oder die zweite Verbindungsstruktur bei Aufbringung einer Kraft, die den vorbestimmten Schwellenwert überschreitet, mindestens zeitweise verformbar ist, wodurch die erste und die zweite Verbindungsstruktur losgelöst werden und ein Lösen der Verbindung zwischen der Patientenschnittstelle (108) und dem Verbinder (104) ermöglicht wird, ohne eine strukturelle Beschädigung, beispielsweise ein Brechen oder Zerbrechen, an der ersten und/oder der zweiten Verbindungsstruktur zu verursachen.

8. Patientenschnittstellenanordnung (100) nach einem vorhergehenden Anspruch, wobei die Patientenschnittstelle (108) eine Sauerstoffzufuhrmaske (102) umfasst.

9. Patientenschnittstellenanordnung (100) nach einem vorhergehenden Anspruch, wobei die erste Verbindungsstruktur mindestens einen Abschnitt einer inneren Oberfläche der Patientenschnittstelle (108) umfasst und wobei die zweite Verbindungsstruktur eine äußere Oberfläche des Verbinders (104) umfasst.

10. Patientenschnittstellenanordnung (100) nach einem vorhergehenden Anspruch, wobei die zweite Verbindungsstruktur mindestens einen Vorsprung (116,118) umfasst, der sich von einem Hauptkörper und/oder Arm des Verbinders (104) nach außen erstreckt.

11. Patientenschnittstellenanordnung (100) nach Anspruch 10, wobei der mindestens eine Vorsprung (116,118) eine Widerlageroberfläche zum Eingreifen in eine entsprechende Widerlageroberfläche der ersten Verbindungsstruktur umfasst und wobei sich der mindestens eine Vorsprung (116,118) mindestens teilweise ringförmig um einen Hauptkörper und/oder Arm des Verbinders (104) herum erstreckt.

12. Patientenschnittstellenanordnung (100) nach einem vorhergehenden Anspruch, wobei die erste und die zweite Verbindungsstruktur im Gebrauch drehbar verbunden sind.

13. Patientenschnittstellenanordnung (100) nach einem vorhergehenden Anspruch, wobei die Patientenschnittstelle (108) und/oder der Verbinder eine Anschlagstruktur umfasst, um ein zu weites Einführen des Verbinders (104) in die Patientenschnittstelle (108) im Gebrauch zu verhindern /oder um eine Bewegung des Verbinders (104) relativ zur Patientenschnittstelle (108) entlang einer Längsachse des Verbinders (104) im Gebrauch zu verhindern.

## Revendications

1. Ensemble interface de patient (100) comprenant une interface de patient (108), un connecteur (104) destiné à raccorder l'interface de patient à un conduit (106), le conduit (106) étant relié de manière non libérable au connecteur (104), l'interface de patient (108) comprenant une première formation de raccord et le connecteur (104) comprenant une seconde formation de raccord, la première formation de raccord comprenant une fente (112) destinée à recevoir au moins une partie de la seconde formation de raccord, ladite fente (112) s'étendant dans une direction qui nécessite l'insertion de la seconde formation de raccord dans la fente (112) dans une direction qui est sensiblement orthogonale à un axe longitudinal du conduit (106), l'interface de patient (108) et le connecteur (104) pouvant être raccordés de sorte que, dans une configuration raccordée, la première formation de raccord et la seconde formation de raccord puissent se mettre en prise l'une dans l'autre, avec un ajustement par encliquetage, pour fournir un raccord non libérable, et
une zone nasale de l'interface de patient (108) s'étendant vers l'avant du reste de l'interface de patient (108), définissant ainsi un étage nasal (110), et ladite fente (112) étant formée dans le corps de masque 108 légèrement au-dessus de l'étage (110), la fente (112) étant définie par au moins une paroi interne (113) de l'interface de patient (108) et la paroi d'étage nasal (110) de l'interface de patient (108).

2. Ensemble interface de patient (100) selon la revendication 1, ladite fente (112) étant formée dans une surface externe de l'interface de patient (108).

3. Ensemble interface de patient (100) selon la revendication 1 ou 2, ladite au moins une paroi interne (113) de l'interface de patient (108) empêchant le retrait du connecteur (104) de la fente (112) dans une direction orthogonale à un axe longitudinal du conduit (106) dans la configuration raccordée, et ladite paroi d'étage nasal (110) de l'interface de patient (108) empêchant le retrait du connecteur (104) de la fente (112) dans une direction sensiblement parallèle à un axe longitudinal du conduit (106) dans la configuration raccordée.

4. Ensemble d'interface de patient (100) selon une quelconque revendication précédente, ledit raccord étant libérable uniquement lors de l'application d'une force qui dépasse une force seuil prédéfinie le long d'un axe de mise en prise entre l'interface de patient (108) et le connecteur (104).

5. Ensemble d'interface de patient (100) selon la revendication 4, ledit raccord étant libérable uniquement lors de l'application d'une force, le long d'un axe de mise en prise entre l'interface de patient (108) et le connecteur (104), possédant une amplitude supérieure ou égale à 20N, supérieure ou égale à 30 N, supérieure ou égale à 40 N, supérieure ou égale à 50 N, supérieure ou égale à 60 N, supérieure ou égale à 70 N, supérieure ou égale à 80 N ou supérieure ou égale à 90 N.

6. Ensemble interface de patient (100) selon une quelconque revendication précédente, ledit raccord étant non libérable au point où le raccord ne peut pas être détaché sans causer de dommages structurels à au moins l'une des première et seconde formations de raccord.

7. Ensemble interface de patient (100) selon la revendication 4 ou la revendication 5, ladite première et/ou ladite seconde formation de raccord étant au moins temporairement déformable lors de l'application d'une force dépassant le seuil prédéfini, séparant ainsi les première et seconde formations de raccord et permettant la libération du raccord entre l'interface de patient (108) et le connecteur (104), sans causer de dommages structurels, par exemple une rupture ou une fracturation, de la première et/ou de la seconde formation de raccord.

8. Ensemble interface de patient (100) selon une quelconque revendication précédente, ladite interface de patient (108) comprenant un masque de distribution d'oxygène (102).

9. Ensemble interface de patient (100) selon une quelconque revendication précédente, ladite première formation de raccord comprenant au moins une partie d'une surface interne de l'interface de patient (108), et ladite seconde formation de raccord comprenant une surface externe du connecteur (104).

10. Ensemble interface de patient (100) selon une quelconque revendication précédente, ladite seconde formation de raccord comprenant au moins une saillie (116, 118) qui s'étend vers l'extérieur à partir d'un corps principal et/ou d'un bras du connecteur (104).

11. Ensemble interface de patient (100) selon la revendication 10, ladite au moins une saillie (116, 118) comprenant une surface de butée destinée à se mettre en prise avec une surface de butée correspondante de la première formation de raccord, et ladite au moins une saillie (116, 118) s'étendant au moins partiellement de manière annulaire autour d'un corps principal et/ou d'un bras du connecteur (104).

12. Ensemble interface de patient (100) selon une quelconque revendication précédente, lesdites première et seconde formations de raccord étant raccordées de manière rotative, lors de l'utilisation.

13. Ensemble interface de patient (100) selon une quelconque revendication précédente, ladite interface de patient (108) et/ou ledit connecteur comprenant une formation de butée destinée à empêcher une insertion excessive du connecteur (104) dans l'interface de patient (108) lors de l'utilisation, et/ou à empêcher le mouvement du connecteur (104) par rapport à l'interface de patient (108), le long d'un axe longitudinal du connecteur (104), lors de l'utilisation.
